# EUROPEAN PATENT APPLICATION

(11) **EP 3 311 879 A1**
(43) Date of publication of application: **25.04.2018**
(21) Application number: 17197054.4
(22) Date of filing: 18.10.2017
(51) Int. Cl.: A61N 1/05, A61N 1/36, A61N 1/372

(54) **SYSTEM AND METHOD USING GESTURES TO CONTROL ELECTROTHERAPY DELIVERY FROM AN ELECTROTHERAPY DEVICE**

(30) Priority: 20.10.2016 US 201662410440 P
(71) Applicant: BIOTRONIK SE & Co. KG, 12359 Berlin (DE)
(72) Inventor: Grosskopf, Rainer Jörg, Portland, OR Oregon 97239 (US); Reinert, Michael, Tigard, OR Oregon 97223 (US)
(74) Representative: Keck, Hans-Georg

(57) **Abstract**

The invention relates to a system (1) for wireless control of an implantable stimulation device (100), wherein the system (1) comprises an implantable stimulation device (100) for delivering electrical stimulation to a patient and an external control device wherein the external control device (200, 300, 400) is configured to transmit a control signal (C) to the stimulation device when the external control device (200, 300, 400) detects a change in translational (T) and/or rotational (R) motion, or a contactless interaction (G) of the patient with the external control device (200, 300, 400). Furthermore, the present invention relates to a corresponding method.

## Description

The present invention relates to a system and method using gestures to control electrotherapy delivery from an electrotherapy device, particularly for providing spinal cord stimulation (SCS).

Spinal cord stimulators provide electrotherapy to reduce pain in patients. Patients frequently adjust the therapy or stimulation parameters in response to their current activities (e.g. sitting, standing, driving, walking, sleeping etc.).

US 2011/0270358 describes programming a spinal cord stimulation system using a gesture-based control done by a physician during programming of parameter settings for the patient. Further, WO 2013/011483 A2 describes a remote control for a medical device, particularly a hearing aid, based on tags (e.g. RFID tags).

Based on the above, the problem addressed by the present invention is to provide a system and method of the afore-mentioned kind having an improved handling.

This problem is solved by a system having the features of claim 1. Preferred embodiments of these aspects of the present invention are stated in the corresponding sub-claims and are described below.

According to claim 1, a system for wireless control of an implantable stimulation device is disclosed, wherein the system comprises an implantable stimulation device for delivering electrical stimulation to a patient and an external control device wherein the external control device is configured to transmit a control signal to the stimulation device when the external control device detects
- a change in translational and/or rotational motion, or
- a contactless interaction of the patient with the external control device.

Particularly the stimulation device is formed as an implantable pulse generator (IPG).

Further, according to an embodiment of the system according to the invention, the stimulation device is configured for spinal cord stimulation, wherein the stimulation device comprises a plurality of electrodes, wherein the stimulation device is configured to deliver spinal cord stimulation via said electrodes.

According to an embodiment of the present invention, the stimulation device may comprise one or even two elongated flexible carriers, wherein each carrier comprises a plurality of electrodes at a distal portion via which SCS may be applied to the spinal cord. Particularly, electrodes of one carrier may act as anodes wherein electrodes of the other carrier may act as cathodes. The two carriers particularly extend along one another.

Further, according to an embodiment of the system according to the invention, said contactless interaction with said external control device (e.g. smartphone) is a motion gesture of the patient in front of the external control device which in this case is particularly configured to detect said gesture e.g. by means of a camera integrated into the external control device or by means of a further sensor connected to the hand that communicates with the external control device.

Particularly, according to an embodiment of the present invention, the external control device is one of or comprises at least one of: a hand-held device, a smartphone, a smartwatch, an object configured to be worn by the patient, a bracelet, a ring.

Particularly, in an embodiment, the external control device (e.g. smartphone), has a software installed that provides a control interface to the stimulation device via a radio connection (e.g. Bluetooth), wherein the external control device further comprises a magnetic field sensor that senses the orientation of the external control device relative to the earth's magnetic field such that when the external control device is rotated by the patient (e.g. when lying on a surface) a corresponding rotation angle is translated into an increase or a decrease of a parameter (e.g. stimulation amplitude) of the electrical stimulation of the stimulation device depending on the direction of the rotation (i.e. the external control device can be used like a rotary knob).

Further, in an embodiment, the system further comprises one or several rings configured to be arranged on fingers of the patient, respectively, which ring(s) are configured to be connected to the external control device (e.g. smart phone) via a radio connection (e.g. Bluetooth), wherein the respective ring comprises a motion sensor, and wherein the external control device (e.g. smartphone) has a software installed that acts as control interface to the stimulation device via a radio connection (e.g. Bluetooth) such that when the patient performs (e.g. taps) a specific finger pattern or movement that is detected by the ring(s), a parameter of the electrical stimulation generated by the stimulation device is changed accordingly.

Furthermore, in an embodiment, the external control device (e.g. smartphone), has a software installed that provides a control interface to the stimulation device via a radio connection (e.g. Bluetooth), wherein the external control device (e.g. smartphone) further comprises a camera that senses a specific movement of the patient (e.g. of its hand) that is translated to a corresponding adjustment of a parameter (e.g. stimulation amplitude) of the electrical stimulation of the stimulation device.

Furthermore, in an embodiment, the external control device is formed as a smartwatch that has a software installed that provides a control interface to the stimulation device via a radio connection (e.g. Bluetooth), wherein the smartwatch comprises a motion sensor that senses a specific movement of the patient (e.g. of its wrist) that is translated to a corresponding adjustment of a parameter (e.g. stimulation amplitude) of the electrical stimulation of the stimulation device.

Particularly, in the framework of the present invention, a smartphone is a cell phone that comprises a display, particularly in the form of a touchscreen, and a microprocessor, and is capable of executing software applications that are installed in a memory on the phone. Likewise, particularly, a smartwatch is a watch that comprises a display, particularly in the form of a touchscreen, and a microprocessor, and is capable of executing software applications that are installed in a memory on the watch. Moreover, the smartphone may maintain a direct or indirect wireless communication link to the stimulation device.

Particularly, instead of a smartwatch, also a bracelet can be used which then comprises said motion sensor configured to detect complex finger gestures or wrist movements.

Furthermore, the external control device can also be formed by one or several pressure - sensitive finger rings that are configured to detect gestures of the hand of the patient wearing said ring(s).

Further, according to an embodiment of the system according to the invention, one of the following parameters of the electrical stimulation is adjusted by means of said external control device:
- stimulation amplitude
- stimulation pulse width
- stimulation frequency and/or stimulation cycles
- electrode or electrodes for stimulation from a plurality of electrodes, e.g. selection of one or more electrodes from an array of electrodes.

A further aspect of the present invention relates to a method for wireless control of an implantable stimulation device using an external control device, wherein
- a change in translational and/or rotational motion of said external control device, or
- a contactless interaction of the patient with said external control device
   is detected by the external control device, and a corresponding control signal is transmitted by the external control device to the stimulation device for controlling the stimulation device.

Particularly, the method according to the invention uses a system according to the present invention as described herein.

Particularly, according to an embodiment of the method according to the present invention, said contactless interaction with said external control device is a motion gesture of the patient in front of the external control device.

Particularly, according to an embodiment of the method according to the present invention, one of the following parameters is adjusted by means of said external control device:
- stimulation amplitude
- stimulation pulse width
- stimulation frequency and/or stimulation cycles
- electrode or electrodes for stimulation from a plurality of electrodes, e.g. selection of one or more electrodes from an array of electrodes.

Furthermore, particularly, the radio communication between the external control device and the stimulation device and between other component such as ring (see above) and the external control device are particularly conducted in the range from 2,402 GHz to 2,480 GHz (e.g. using Bluetooth).

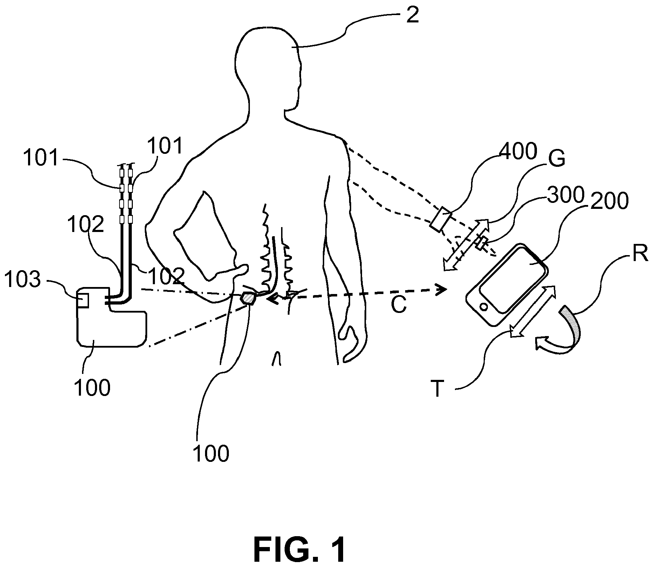

Further features and advantages of the present invention and embodiments thereof shall be explained below with reference to the Figure, wherein
- Fig. 1: shows a schematic representation of a system according to the present invention for wireless control of a electrotherapy device, particularly of a system for spinal cord stimulation.

Fig. 1 shows embodiments of a system 1 for wireless control of a stimulation device 100, here e.g. for spinal cord stimulation according to the present invention. The invention can also be applied to other electrotherapy devices.

Usually, patients 2 use a component 200 such as a remote control (e.g. in the form of a smartphone) throughout the day in order to adjust proper stimulation parameters for the spinal cord stimulation (SCS) for activities such as sitting (e.g. at a desk), or walking, or driving a car. Also stimulation during the day may be different from stimulation during night time etc. For operating an SCS stimulation device 100 patients may use a component 200 such as a smartphone 200 or an actual remote control when less familiar with a touch screen/ smartphone. However, in certain situations handling and operating of such operating means may difficult and cumbersome.

Here, the present invention allows a gesture or movement-based operating of the stimulation device that can be accomplished by the patient in an easy manner.

For this, as shown in Figs 1, the system 1 comprises a stimulation device 100 for delivering SCS having a plurality of electrodes 101, wherein the stimulation device 100 is configured to deliver spinal cord stimulation via said electrodes 101 according to one or several parameters that can be adjusted by the patient.

Particularly, the stimulation device 100 may comprise two flexible carriers 102 for carrying said electrodes 101, which carriers 102 may get tunneled during implantation to the vicinity of the stimulation device (e.g. an implantable pulse generator or IPG) 100 that is typically implanted subcutaneously in the patient's lower abdominal or gluteal region. The carriers 102 may terminate proximally in connectors that are then inserted into the IPG 100 header to allow conducting electrical charge to the electrodes 101. However, any other stimulation device design may also be used.

Now, according to the present invention, as indicated in Fig. 1, the external control device used in the present invention can be a smartphone 200 used alone or in conjunction with one or several finger rings 300, or a bracelet or smartwatch 400. Further, the external control device may also be formed by one or several finger rings 300 (without the help of a device such as a smartphone 200).

Particularly the external control device 200, 300, 400 of the respective embodiment is configured to transmit a control signal C to the stimulation device 100 that can be received by corresponding unit 103 of the stimulation device 100 when the external control device detects a change in translational T and/or rotational R motion, or detects a contactless interaction G, e.g. a gesture G, that the patient forms with a hand.

Particularly, in an embodiment indicated in Fig. 1 the external control device is formed as a smartphone 200 that provides a control interface to the stimulation device 100 via a radio connection (e.g. Bluetooth) C, wherein the external control device 200 further comprises a magnetic field sensor that senses the orientation of the external control device 200 relative to the earth's magnetic field such that when the external control device undergoes a rotation R initiated by the patient when e.g. lying on a surface, a corresponding rotation angle is coded as a control signal C and transmitted to the stimulation device 100 which adjusts a selected parameter of the electrical stimulation accordingly.

Further, in an alternative embodiment shown in Fig. 1, the system 1 may further comprise one or several rings 300 configured to be arranged on fingers of the patient 2, respectively, which ring(s) 300 are configured to be connected to the external control device (e.g. smart phone) 200 via a radio connection (e.g. Bluetooth), wherein the respective ring 300 comprises a motion sensor, and wherein said smartphone 200 again acts as control interface to the stimulation device 100 via a radio connection (e.g. Bluetooth) C such that when the patient performs (e.g. taps) a specific finger pattern or movement that is detected by the ring(s) 300, a corresponding control signal C is transmitted from the smartphone 200 to the stimulation device 100 which adjusts a selected parameter of the electrical stimulation generated by the stimulation device 100 accordingly.

Furthermore, in a further embodiment, the smartphone 200 may also be configured to detect a specific motion or gesture of a patient's hand and/or fingers by means of a camera. Also here said gesture is transformed into a corresponding control signal C that is transmitted to the stimulation device 100 and lead to a corresponding adjustment of a selected parameter of the electrical stimulation applied by the stimulation device 100.

Furthermore, in an alternative embodiment shown in Fig. 1, the external control device can formed as a smartwatch 400 that has a software installed that provides a control interface to the stimulation device 100 via a radio connection (e.g. Bluetooth) C, wherein the smartwatch 400 comprises a motion sensor that senses a specific movement of the patient (e.g. of its wrist) that is then translated into a corresponding control signal C which transmitted to the stimulation device 100 which in turn adjustment a selected parameter of the electrical stimulation applied by the stimulation device 100 accordingly.

In a further embodiment, instead of a smartwatch 400, also a bracelet 400 can be used which then comprises said motion sensor configured to detect complex finger gestures G or wrist movements.

Furthermore, the external control device can also be formed by one or several pressure - sensitive finger rings 300 that are configured to detect gestures G of the hand of the patient wearing said ring(s) 300. Here, a further device such as a smartphone 200 can be omitted and the ring(s) may be configured to transmit a corresponding control signal C to unit 103 of the stimulation device 100 which in turn adjusts the stimulation parameter in question using said control signal C.

For example, the following parameters may be identified by certain gestures G and or movements T, R and particularly adjusted by these gestures G or movements T, R:
- stimulation amplitude
- stimulation pulse width
- stimulation frequency and/or stimulation cycles
- electrode or electrodes for stimulation from a plurality of electrodes, e.g. selection of one or more electrodes from an array of electrodes.

Thus, to summarize, the present invention particularly allows the individual patient or caregiver to configure the system 1 to associate gestures/movements with changes in therapy delivery parameters.

In this way the user interaction is reduced from having to push several buttons on the remote control device to simply performing a gesture or certain movement.

## Claims

1. A system (1) for wireless control of an implantable stimulation device (100), wherein the system (1) comprises an implantable stimulation device (100) for delivering electrical stimulation to a patient and an external control device wherein the external control device (200, 300, 400) is configured to transmit a control signal (C) to the stimulation device when the external control device (200, 300, 400) detects
- a change in translational (T) and/or rotational (R) motion, or
- a contactless interaction (G) of the patient with the external control device (200, 300, 400).

2. The system according to claim 1, **wherein** the stimulation device (100) is configured for spinal cord stimulation, wherein the stimulation device (100) comprises a plurality of electrodes (101), wherein the stimulation device is configured to deliver spinal cord stimulation via said electrodes (101).

3. The system according to claim 1 or 2, **wherein** said contactless interaction (G) with said external control device is a motion gesture of the patient (2) in front of the external control device (200, 300).

4. The system according to one of the preceding claims, **wherein** the external control device is one of: a hand-held device (200), a smartphone (200), a smartwatch (400), an object configured to be worn by the patient, a bracelet (400), a ring (300).

5. The system according to one of the preceding claims, **wherein** one of the following parameters of the electrical stimulation is adjusted by means of said control signal (C):
- stimulation amplitude
- stimulation pulse width
- stimulation frequency and/or stimulation cycles
- electrode or electrodes for stimulation from a plurality of electrodes.

6. A method for wireless control of an implantable stimulation device (100) using an external control device (200, 300, 400), wherein
- a change in translational (T) and/or rotational (R) motion of said external control device (200, 400), or
- a contactless interaction (G) of the patient with said external control device (200) is detected by the external control device, and a corresponding control signal is transmitted by the external control device to the stimulation device for controlling the stimulation device.

7. The method according to claim 6, **wherein** the stimulation device is configured for spinal cord stimulation, wherein the stimulation device (100) comprises a plurality of electrodes (101), wherein the stimulation device is configured to deliver spinal cord stimulation via said electrodes (101).

8. The method according to claim 6 or 7, **wherein** said contactless interaction (G) with said external control device (200) is a motion gesture of the patient in front of the external control device.

9. The method according to one of the claims 6 to 8, **wherein** the external control device is one of: a hand-held device (200, 400), a smartphone (200), a smartwatch (400), an object configured to be worn by the patient, a bracelet (400), a ring (300).

10. The method according to one the claims 6 to 9, **wherein** one of the following parameters is adjusted by means of said control signal:
- stimulation amplitude
- stimulation pulse width
- stimulation frequency and/or stimulation cycles
- electrode or electrodes for stimulation from a plurality of electrodes.
